# FASCICULE DE BREVET EUROPEEN

(11) **EP 2 102 202 B1**
(45) Date de publication et mention de la délivrance du brevet: **17.07.2013**
(21) Numéro de dépôt: 07871783.2
(22) Date de dépôt: 03.12.2007
(51) Int. Cl.: C07D 471/04, A61K 31/4375, A61P 1/00

(54) **CONGLOMERATS DE SELS DE POTASSIUM DU TENATOPRAZOLE**
TENATOPRAZOL-KALIUMSALZ-GEMISCHE
CONGLOMERATES OF TENATOPRAZOLE POTASSIUM SALTS

(30) Priorité: 04.12.2006 FR 0610553
(43) Date de publication de la demande: 23.09.2009
(73) Titulaire: Sidem Pharma SA, 2613 Luxembourg (LU)
(72) Inventeur: COHEN, Avraham, Tel Aviv (IL); SCHUTZE, François, F-78860 Saint-Nom-La-Breteche (FR); CHARBIT, Suzy, F-94000 Creteil (FR); BERNAD, Stéphane, F-94210 La Varenne Saint-Hilaire (FR); TAUVEL, Guillaume, F-76000 Rouen (FR); PETIT, Marie-Noëlle, F-76130 Mont Saint-Aignan (FR); COQUEREL, Gérard, F-76520 Boos (FR)
(74) Mandataire: L'Helgoualch, Jean
(86) Numéro de dépôt international: PCT/FR2007/001981
(87) Numéro de publication internationale: WO 2008/081104

(56) Documents cités:
- EP-A1- 0 254 588
- WO-A-2004/074285
- WO-A-2006/043280
- FR-A1- 2 871 800

## Description

La présente invention concerne les sels de ténatoprazole, et plus particulièrement des sels de potassium du ténatoprazole cristallisant sous forme de conglomérats, ainsi que leur utilisation pour former un sel de sodium de l'un ou l'autre des énantiomères du ténatoprazole.

Le ténatoprazole, ou 5-méthoxy-2-[[(4-méthoxy-3,5-diméthyl-2-pyridyl)méthyl]sulfinyl]imidazo[4,5-b]pyridine, décrit dans le brevet EP 254 588, fait partie des médicaments considérés comme des inhibiteurs de la pompe à protons ayant pour effet d'inhiber la sécrétion gastrique, et il peut être utilisé plus particulièrement dans le traitement du reflux gastro-oesophagien, des hémorragies digestives et des dyspepsies.

Il peut être représenté par la formule générale suivante :

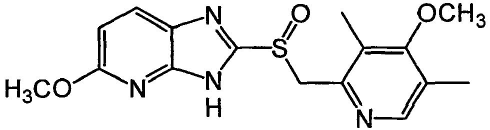

Ce composé présente une asymétrie au niveau de l'atome de soufre et peut donc se présenter sous forme de racémique, ou sous forme de l'un ou l'autre des deux énantiomères. Ces énantiomères, comme le racémique, peuvent classiquement être utilisés sous forme de sels, tels que les sels de magnésium, de potassium ou de sodium, qui sont généralement plus faciles à manipuler que la forme acide correspondante.

Des travaux récents ont montré que, contrairement à tous les autres inhibiteurs de la pompe à protons tels que, par exemple, l'oméprazole ou le lansoprazole, le ténatoprazole possède une durée d'action nettement prolongée, résultant d'une demi-vie plasmatique environ 7 fois supérieure. Ainsi, les données médicales recueillies montrent que le ténatoprazole apporte un niveau de soulagement des symptômes et de cicatrisation des lésions gastriques supérieur à celui des autres médicaments connus appartenant à la même classe thérapeutique des inhibiteurs de la pompe à protons, ce qui permet alors son utilisation efficace dans le traitement des symptômes atypiques et oesophagiens du reflux gastro-oesophagien, des hémorragies digestives et des dyspepsies, comme indiqué ci-dessus.

De plus, il a été montré que chacun des énantiomères (+) et (-), ou R et S respectivement, contribue de manière différente aux propriétés du ténatoprazole, et que le S-ténatoprazole a des propriétés pharmacocinétiques significativement différentes de celles du racémique et de l'autre énantiomère de configuration R.

En particulier, les travaux faisant l'objet du brevet EP 1 664 044 ont montré que le sel de sodium monohydraté du S-ténatoprazole présente des propriétés inattendues qui le distinguent du S-ténatoprazole lui-même, et des autres inhibiteurs de la pompe à protons, et plus particulièrement une excellente solubilité facilitant les modalités de mise sous forme pharmaceutique et améliorant significativement l'absorption et l'efficacité thérapeutique du médicament le contenant. WO 2006/043280 décrit des sels de potassium du ténatoprazole racémique.

La demande WO 2004/074285 décrit le R-ténatoprazole et un procédé pour sa préparation.

Le sel de sodium du S-ténatoprazole décrit dans le brevet EP 1 664 044 peut être préparé par deux procédés distincts.

Le premier procédé de préparation consiste à faire agir de l'hydroxyde de sodium sur le S-ténatoprazole obtenu par résolution du racémique, à une température comprise entre 50 et 70°C, puis à faire précipiter le sel obtenu après élimination du solvant. -

Le deuxième procédé consiste succinctement à effectuer une oxydation énantiosélective du sulfure correspondant au moyen d'un agent oxydant, en présence d'un catalyseur à base de vanadium ou de titane et d'un ligand chiral approprié, puis à effectuer une salification par l'hydroxyde de sodium.

La mise en oeuvre de l'un ou l'autre de ces procédés donne des résultats satisfaisants en termes d'excès énantiomérique et de rendement. Toutefois, il demeure nécessaire de trouver de nouvelles voies de synthèse des énantiomères du ténatoprazole, qui non seulement procurent d'excellents résultats en ce qui concerne le rendement et la sélectivité, mais, en outre, utilisent une quantité minimale de substance toxique, voire aucune substance toxique, afin de limiter les risques liés à l'emploi de substances nuisibles à la santé et à l'environnement.

Les inventeurs ont ainsi cherché à obtenir des composés à partir desquels de nouvelles voies de synthèse, répondant aux critères mentionnés ci-dessus, étaient envisageables.

Des essais intensifs leur ont permis de mettre au point de nouveaux sels de potassium du ténatoprazole, se présentant sous une forme cristalline particulière, et de montrer que ces sels pouvaient être utilisés de façon avantageuse pour la préparation du sel de sodium des énantiomères de configuration S et R du ténatoprazole, dont les avantages thérapeutiques ont été décrits plus haut.

L'invention a donc pour objet l'utilisation des sels de potassium du ténatoprazole, représentés par la formule générale suivante:

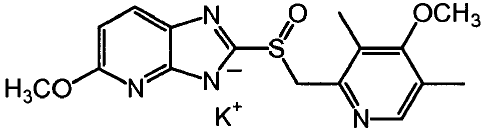

Ces sels sont constitués d'un mélange équimolaire d'énantiomères de configuration R et S, cristallisant sous forme de conglomérats, lesdits sels étant choisis parmi le sel de potassium dihydraté du ténatoprazole, le diméthanolate de potassium du ténatoprazole, le diéthanolate de potassium du ténatoprazole et l'éthylène-glycolate de potassium du ténatoprazole.

On entend par "conglomérat" un mélange racémique qui, dans un solvant donné et dans une certaine plage de températures, est constitué de deux types de cristaux ne contenant chacun que des molécules de même configuration, et incorporant ou non des molécules de solvant. Si des molécules de solvant sont incorporées, on parle alors de "solvates". Les hydrates sont des solvates où le solvant est l'eau.

La cristallisation des sels de potassium du ténatoprazole de l'invention sous forme de conglomérat signifie que l'empilement cristallin permet une discrimination spontanée des énantiomères S et R lors de la cristallisation. Cependant, il faut noter qu'il est impossible de prédire si un mélange racémique va cristalliser sous forme de conglomérat, et il est impossible de prévoir le type d'empilement d'une phase. On sait aussi que 5% environ des mélanges racémiques cristallisent sous forme de conglomérats (C.P. Brock et al., Chem. Mater., 1994, 6, p.1118-1127). Ainsi, le ténatoprazole sous forme acide cristallise sous forme de racémique, de même que ses sels de sodium.

Il a également été montré que l'obtention d'un conglomérat est une conditions nécessaire mais pas suffisante pour un dédoublement par cristallisation préférentielle (G. Coquerel, Preferential Crystallization in Topic in Current Chemistry, Novel Optical Resolution Technologies, Springer, Berlin- Heidelberg, Eds K. Sakai, N. Hirayama and R. Tamura, 2007, 269, 1-51).

Les caractéristiques des composés selon l'invention sont spécifiées ci-après.

Lorsque le sel est constitué du sel de potassium dihydraté du ténatoprazole, il produit un spectre de diffraction aux rayons X comprenant des raies aux distances inter-réticulaires suivantes : 15,16 ; 8,07 ; 7,59 ; 7,23 ; 7,02 ; 5,72 ; 5,55 ; 5,41 ; 5,32 ; 5,05 ; 4,51 ; 4,35 ; 4,29 ; 4, 22 ; 4, 061 ; 3, 926 ; 3, 748 ; 3, 624 ; 3, 539 ; 3,487 ; 3, 436 ; 3, 392 ; 3, 292 ; 3, 252 ; 3, 222 ; 3, 102 ; 3,034 (en Angströms).

Lorsque le sel est un solvate constitué du diméthanolate de potassium du ténatoprazole, il produit un spectre de diffraction aux rayons X comprenant des raies aux distances inter-réticulaires suivantes : 13,79 ; 6,87 ; 5,84 ; 5,44 ; 4,83 ; 4,57 ; 4,31 ; 3,97 ; 3,921 ; 3,865 ; 3,794 ; 3,644 ; 3,56 ; 3,508 ; 3,431 ; 3,393 ; 3,309 ; 3,233 ; 3,116 (en Angströms).

Lorsque le sel est un solvate constitué du diéthanolate de potassium du ténatoprazole, il produit un spectre de diffraction aux rayons X comprenant des raies aux distances inter-réticulaires suivantes : 13,79 ; 10,73 ; 9,41 ; 7,75 ; 6,87 ; 6,71 ; 6,40 ; 6,25 ; 5,97 ; 5,463 ; 5,377 ; 5,128 ; 4,918 ; 4,711 ; 4,562 ; 4,418 ; 4,020 ; 3,936 ; 3,892 ; 3,855 ; 3,705 ; 3,656 ; 3,623 ; 3,573 ; 3,508 ; 3,460 ; 3,446 ; 3,363 ; 3,302 ; 3,249 ; 3,167 ; 3,129 ; 3,102 ; 3,043 (en Angströms).

Lorsque le sel est un solvate constitué de l'éthylène glycolate de potassium du ténatoprazole, il produit un spectre de diffraction aux rayons X comprenant des raies aux distances inter-réticulaires suivantes : 12,92 ; 9,30 ; 7,89 ; 7,59 ; 6,77 ; 6,05 ; 5,828 ; 5,604 ; 5,409 ; 5,026 ; 4,907 ; 4,657 ; 4,569 ; 4,478 ; 4,295 ; 4,178 ; 4,013 ; 3,972 ; 3,931 ; 3,798 ; 3,692 ; 3,650 ; 3,608 ; 3,543 ; 3,396 ; 3,340 ; 3,300 ; 3,271 ; 3,204 ; 3,148 ; 3,105 ; 3,060 ; 3,013 (en Angströms).

Un objet de la présente invention est l'utilisation d'un tel sel de potassium pour la préparation d'un sel de sodium du ténatoprazole énantiomériquement pur.

La préparation du sel de sodium du ténatoprazole énantiomériquement pur est avantageusement réalisée par dédoublement des énantiomères optiques d'un des sels de potassium racémique, selon un procédé de cristallisation préférentielle, puis par transformation de l'un desdits énantiomères optiques du sel de potassium en l'énantiomère correspondant du sel de sodium.

Un procédé de cristallisation préférentielle est notamment décrit dans le brevet FR 2 710 337. Ce procédé permet la cristallisation alternée des deux composés chiraux R et S à partir du conglomérat thermodynamiquement stable correspondant. Cette technique présente de nombreux avantages :
- elle évite l'utilisation d'un agent chiral intermédiaire, dont la synthèse et la récupération rendent le procédé plus complexe ;
- les deux antipodes optiques sont chacun obtenus directement ;
- le rendement peut être considéré comme quantitatif en chaque énantiomère du fait des recyclages successifs des solutions mères ;
- la purification des cristaux d'énantiomères bruts obtenus est aisée.

Dans le cas du ténatoprazole, ce procédé consiste de façon générale à mettre en oeuvre les étapes successives suivantes :
(i) dissolution du ténatoprazole acide racémique et de potasse KOH dans un solvant ou dans un mélange de solvants choisis parmi l'eau, le méthanol, l'éthanol ou l'éthylène glycol. Lors de cette étape et à cause de la solubilité non congruente de ces sels, il est préférable d'utiliser un excès de KOH par rapport au nombre d'équivalent de ténatoprazole, par exemple un excès compris entre 10 et 30% ;
(ii) cristallisation du sel de potassium soit par évaporation du solvant, soit par baisse de la température du système, de sorte à obtenir le sel de potassium du ténatoprazole sous forme de conglomérat ;
(iii) détermination de la température d'homogénéisation du mélange racémique appelée T_{L} ;
(iv) ajout d'une faible quantité de ténatoprazole acide énantiomériquement pur avec de la potasse ;
(v) détermination de la température d'homogénéisation du système appelée T_{HOMO};
(vi) choix de la température initiale du système, appelée T_{I}, pour le début du procédé de dédoublement, de sorte que T_{I} > T_{HOMO} ;
(vii) diminution de la température jusqu'à la température de fin de dédoublement, appelée T_{F}, soit par application d'une rampe de température, soit par une trempe ;
(viii) ajout du sel de potassium énantiomériquement pur choisi, à la température appelée T_{E}, de sorte à ensemencer le système. La quantité de sel ajouté est de l'ordre de 1% de la récolte attendue. Lorsque la diminution de température de l'étape (vii) a été réalisée par application d'une rampe de température, on a T_{HOMO} > T_{E} ≥ T_{F}. Lorsque la diminution de température a été réalisée par une trempe, on a T_{E} = T_{F}.

Au cours du refroidissement, l'énantiomère en excès cristallise (par un double mécanisme de germination secondaire et de croissance), alors que l'énantiomère en défaut accuse un retard à la germination primaire et reste donc sursaturé. L'appauvrissement de la solution est suivi par analyse polarimétrique.
(ix) filtration du système à une température appelée T_{F} = T_{FILTRATION}, lorsque l'antipode optique commence à nucléer, de préférence légèrement avant la germination de l'antipode, autrement dit lorsque la dérivée de la courbe du suivi polarimétrique change de signe.

Par compensation en solvant et en mélange racémique, le système est ensuite placé dans une position symétrique par rapport à la situation initiale, de sorte à être prêt le cas échéant pour la séparation du second énantiomère.

On peut alors répéter des recyclages de liqueur mère et ajouter des masses de mélange racémique équivalentes à celle de l'énantiomère récolté à la filtration précédente. Si la première cristallisation a permis d'isoler l'énantiomère (S), chaque opération impaire et paire donne respectivement les énantiomères (S) et (R). De cette façon, une quantité 2Q de mélange racémique peut être dédoublée en deux masses Q d'énantiomères de très haute pureté car la présence d'un conglomérat stable permet également de purifier de façon optimale les récoltes brutes issues des cristallisations préférentielles.

Le mélange racémique de départ (étape (i)) peut être obtenu par les procédés connus, par exemple suivant le procédé décrit dans le brevet EP 254 588. Ainsi, il peut être préparé en traitant par un agent oxydant, tel qu'un acide perbenzoïque, le sulfure correspondant provenant de la condensation d'un thiol et d'une pyridine. La réaction se fait de préférence en présence d'une base telle que l'hydroxyde de potassium dans un solvant approprié, par exemple l'éthanol, à chaud.

Le sel de potassium énantiomériquement pur ajouté à l'étape (viii) peut être obtenu à partir du mélange racémique, par des techniques bien connues, en utilisant une méthode de séparation appropriée, par exemple par chromatographie préparative sur colonne, comme la chromatographie chirale ou HPLC, puis salification et préparation du solvate correspondant, ou par un procédé tel que décrit dans les exemples 5 à 8.

Un sel de potassium selon l'invention, en raison de ses propriétés inhibitrices de la sécrétion d'acide gastrique, peut être utilisé en tant que tel dans une composition pharmaceutique destinée au traitement des affections gastro-intestinales, et plus particulièrement au traitement des ulcères gastriques et duodénaux, du reflux gastro-oesophagien, des hémorragies digestives et des dyspepsies. Conformément à la présente invention, il peut également être utilisé pour la préparation du sel de sodium du S-ténatoprazole, ou du sel de sodium du R-ténatoprazole.

La transformation de l'un des énantiomères optiques du sel de potassium en l'énantiomère correspondant du sel de sodium peut être faite par des procédés connus, par exemple en utilisant des résines échangeuses d'ion telles que des zéolithes, ou en faisant cristalliser l'acide libre et en salifiant celui-ci par de l'hydroxyde de sodium.

Le sel de sodium du S-ténatoprazole obtenu comme indiqué plus haut peut lui-même être avantageusement utilisé dans la fabrication d'un médicament pour le traitement des pathologies digestives où une inhibition de la secrétion acide doit être intense et prolongée, ou, chez des patients polymédicamentés, pour le traitement des pathologies digestives, du reflux gastro-oesophagien et des hémorragies digestives résistant aux autres inhibiteurs de la pompe à protons.

Il peut également être utilisé pour la fabrication d'un médicament procurant une amélioration significative de la cicatrisation ainsi qu'une augmentation de la vitesse de normalisation des modifications histologiques des lésions gastriques et oesophagiennes chez l'animal ou l'homme, et par conséquent une forte diminution des récidives.

Le sel de sodium du S-ténatoprazole obtenu à partir des composés selon l'invention peut également être utilisé pour la fabrication d'un médicament ayant des propriétés pharmacocinétiques améliorées permettant une posologie d'une seule prise de médicament par jour dans les indications pertinentes, en particulier pour l'éradication d'Helicobacter pylori dans le traitement des ulcères duodénaux, qui nécessitent deux prises, matin et soir, avec les autres inhibiteurs de pompes à protons.

Les exemples suivants sont destinés à illustrer l'invention sans en limiter la portée.

Les exemples 1 à 4 portent sur la préparation des composés racémiques selon l'invention.

Les exemples 5 à 8 portent sur la préparation des composés correspondants énantiomériquement purs, qui sont utilisés pour l'ensemencement du système lors du procédé de dédoublement par cristallisation préférentielle des composés selon l'invention.

Les exemples 9 à 13 décrivent les conditions expérimentales utilisées pour la mise en oeuvre de procédés de dédoublement de composés selon l'invention par cristallisation préférentielle, ainsi que les résultats obtenus. Dans ces exemples, la diminution de température à l'étape (vii) est réalisée par application d'une rampe de température. Les puretés optiques ont été mesurées par polarimétrie à la longueur d'onde λ = 546 nm avec une concentration d'environ 20 mg/L.

L'exemple 14 décrit la structure cristalline de sels de potassium énantiomériquement purs.

Les exemples 15 à 18 sont des exemples comparatifs destinés à montrer que l'utilisation de solvants (tels que l'alcool isopropylique, un mélange éthanol/propylène glycol, un mélange éthanol/eau, ou un mélange N-méthylpyrrolidinone/eau) autres que ceux utilisés dans le procédé selon l'invention (à savoir le méthanol, l'éthanol, l'eau ou l'éthylène glycol) ne donnent pas les résultats attendus, car ils ne permettent pas d'obtenir de cristaux sous forme de conglomérats.

L'exemple 19 porte sur l'utilisation de composés selon l'invention pour la préparation du sel de sodium du ténatoprazole énantiomériquement pur.

### Dispositif expérimental

Le dispositif expérimental utilisé pour les expériences décrites dans les exemples 9 à 12 est identique, à l'exception du réacteur dont la taille et la forme varient en fonction du volume du système.

Les opérations sont effectuées alternativement dans deux tubes d'environ 12 cm de hauteur et de 29 mm de diamètre à col rodé (29/32 n°4) pour l'exemple 9. Pour les exemples 10 et 11, les dimensions des tubes sont d'environ 20 cm de hauteur et 5 cm de diamètre à col rodé (29/32 n°4).

Ces tubes sont munis, dans leur partie supérieure, d'un tube latéral pour établir une dépression nécessaire lors de la filtration. Les cristaux sont récupérés sur verre fritté n°2 ou 3, adaptable sur chaque tube par l'intermédiaire d'un anneau de caoutchouc. L'agitation est assurée par un barreau magnétique. Les liqueurs passent successivement d'un tube à l'autre. Ces transferts, réduits au maximum, n'empêchent pas des pertes entre chaque opération. Plus les quantités de produit utilisé seront faibles, plus les pertes seront proportionnellement grandes. On peut les répertorier en deux catégories:
- pertes, au niveau du verre fritté et dans le tube initial, de liqueur-mère contenant l'excès énantiomérique de fin de cristallisation. La compensation se fait par ajout de cristaux racémiques et de solvant;
- pertes en solvant principalement dues à la filtration créée par dépression. La compensation se fait par ajout à chaque opération de solvant supplémentaire.

Dans certains cas, par exemple lorsque l'on utilise un solvant très volatil, le procédé de compensation doit être plus précis. Une petite quantité de la solution est prélevée, afin d'en déterminer le composition, permettant ensuite une compensation rigoureuse.

Afin d'accéder à une bonne reproductibilité des résultats, le liquide caloporteur circulant dans la double enveloppe est régulé en température avec une précision de ± 0,1°C. L'appareillage mis en oeuvre permet de fixer une loi de refroidissement reproductible.

Dans l'exemple 12, les opérations sont réalisées dans un réacteur de 2 litres à double enveloppe thermostatée. L'agitation est mécanique et est assurée au moyen d'une pale à double hélice. La filtration a lieu au moyen d'une centrifugeuse munie d'une chaussette de 20 cm de diamètre, de 10 cm de hauteur et dont le diamètre des pores du media filtrant en nylon est de 20 µm, la liqueur mère récupérée dans un récipient étant transvasée dans le réacteur.

### Exemple 1

### Préparation des sels de potassium du ténatoprazole racémiques dihydratés

Les exemples 1a à 1c décrivent trois procédés distincts pour la préparation de sels de potassium du ténatoprazole racémiques dihydratés selon l'invention.

### Exemple 1a

On dissout 0,99 g (soit 17,6 mmol) de potasse (KOH) dans 5 mL d'eau. 5,14 g (soit 14,9 mmol) de ténatoprazole acide racémique sont ajoutés à la solution. La dissolution est rapide. Après 12 heures d'agitation à 0°C, un produit cristallisé est filtré sur Büchner. Le produit cristallisé est analysé par diffraction des rayons X sur poudres.

### Exemple 1b

On dissout 0,22 g (soit 3,9 mmol) de potasse dans 30 mL de méthanol, puis 1,13 g (soit 3,3 mmol) de ténatoprazole acide racémique sont ajoutés à la solution. La dissolution est rapide. Après quelques minutes, le produit cristallisé apparaît. Ce produit est filtré sur Büchner, séché puis analysé par diffraction des rayons X sur poudres.

### Exemple 1c

On dissout 0,23 g (soit 4,1 mmol) de potasse dans 30 mL d'éthanol, puis 1,23 g (soit 3,6 mmol) de ténatoprazole acide racémique sont ajoutés à la solution. La dissolution est rapide. Après quelques minutes, le produit cristallisé apparaît. Ce produit est filtré sur Büchner, séché puis analysé par diffraction des rayons X sur poudres.

### Exemple 2

### Préparation des sels de potassium du ténatoprazole racémiques solvatés par le méthanol

On dissout 0,11 g (soit 1,96 mmol) de potasse dans 15 mL de méthanol pur, puis 0,58 g (soit 1,7 mmol) de ténatoprazole acide racémique sont ajoutés à la solution. La dissolution est rapide. Après quelques minutes, le produit cristallisé apparaît. Ce produit est filtré par gravité sur papier filtre puis analysé humide de solution mère par diffraction des rayons X sur poudres en chambre d'atmosphère saturée de méthanol.

### Exemple 3

### Préparation des sels de potassium du ténatoprazole racémiques solvatés par l'éthanol

On dissout 0,23 g (soit 4,1 mmol) de potasse dans 30 mL d'éthanol, puis 1,23 g (soit 3,6 mmol) de ténatoprazole acide racémique sont ajoutés à la solution. La dissolution est rapide. Après quelques minutes, le produit cristallisé apparaît. Ce produit est filtré par gravité sur papier filtre puis analysé humide de solution mère par diffraction des rayons X sur poudres en chambre d'atmosphère saturée d'éthanol.

### Exemple 4

### Préparation des sels de potassium du ténatoprazole racémiques solvatés par l'éthylène glycol

Les exemples 4a et 4b décrivent deux procédés distincts pour la préparation de sels de potassium du ténatoprazole racémiques solvatés par l'éthylène glycol.

### Exemple 4a

On dissout 0,3 g (soit 5,4 mmol) de potasse dans un mélange de solvants composé de 20 mL d'éthanol et de 2 mL d'éthylène glycol, puis 1,54 g (soit 4,5 mmol) de ténatoprazole acide racémique sont ajoutés à la solution. La dissolution est rapide. Après quelques heures d'agitation, le produit cristallisé apparaît. Ce produit est filtré sur Büchner, séché puis analysé par diffraction des rayons X sur poudres.

### Exemple 4b

On dissout 0,2 g (soit 3,6 mmol) de potasse dans un mélange de solvants composé de 10 mL de méthanol et 15 mL d'éthylène glycol, puis 1,03 g (soit 3 mmol) de ténatoprazole acide racémique sont ajoutés à la solution. La dissolution est rapide. Après quelques heures d'agitation à 0°C, le produit cristallisé apparaît. Ce produit est filtré sur Büchner, séché puis analysé par diffraction des rayons X sur poudres.

### Exemple 5

### Préparation des sels de potassium du ténatoprazole énantiomériquement purs dihydratés

Les exemples 5a à 5c décrivent trois procédés distincts pour la préparation de sels de potassium du ténatoprazole énantiomériquement purs dihydratés.

### Exemple 5a

On dissout 0,26 g (soit 0,75 mmol) de ténatoprazole acide de configuration S dans 14 g d'éthanol, en présence de 0,05 g (soit 0,89 mmol) de KOH et 10 gouttes d'eau. Après évaporation partielle du solvant, le produit est filtré sur Büchner et laissé à l'air ; le diéthanolate initialement formé subit une conversion rapide et totale en dihydrate.

### Exemple 5b

On dissout 2,7 g (soit 7,8 mmol) de ténatoprazole acide de configuration S dans 23 g de dioxane, en présence de 1,8 mg d'une solution de potasse à 5,1 mol.L⁻¹ (soit 9,2 mmol). Après évaporation partielle du solvant, le produit est filtré sur Büchner et analysé par diffraction des rayons X sur poudres.

### Exemple 5c

On dissout 0,15 g (soit 0,43 mmol) de ténatoprazole acide de configuration S dans 3,9 g de tétrahydrofurane, en présence de 0,1 mL d'une solution aqueuse de potasse à 4,75 mol.L⁻¹ (soit 0,48 mmol). Après évaporation lente du solvant, le produit est analysé par diffraction des rayons X sur poudres.

### Exemple 6

### Préparation des sels de potassium du ténatoprazole énantiomériquement purs solvatés par le méthanol

On dissout 0,3 g (soit 5,4 mmol) de potasse dans 10 mL de méthanol, puis 1,53 g (soit 4,4 mmol) de ténatoprazole acide de configuration S sont ajoutés à la solution. La dissolution est rapide. Après quelques minutes, le produit cristallisé apparaît. Ce produit est filtré par gravité sur papier filtre puis analysé humide de solution mère par diffraction des rayons X sur poudres en chambre d'atmosphère saturée de méthanol.

### Exemple 7

### Préparation des sels de potassium du ténatoprazole énantiomériquément purs solvatés par l'éthanol

On dissout 0,22 g (soit 3,9 mmol) de potasse dans 30 mL d'éthanol, puis 1,13 g (soit 3,3 mmol) de ténatoprazole acide de configuration S sont ajoutés à la solution. La dissolution est rapide. Après quelques minutes, le produit cristallisé apparaît. Ce produit est filtré par gravité sur papier filtre puis analysé humide de solution mère par diffraction des rayons X sur poudres en chambre d'atmosphère saturée d'éthanol.

### Exemple 8

### Préparation des sels de potassium du ténatoprazole énantiomériquement purs solvatés par l'éthylène glycol

Les exemples 8a à 8c décrivent trois procédés distincts pour la préparation de sels de potassium du ténatoprazole énantiomériquement purs solvatés par l'éthylène glycol.

### Exemple 8a

On dissout 0,09 g (soit 1,6 mmol) de potasse dans un mélange de solvants composé de 3 mL d'éthanol et de 1 mL d'éthylène glycol, puis 0,48 g (soit 1,4 mmol) de ténatoprazole acide de configuration S sont ajoutés à la solution. La dissolution est rapide. Après quelques heures d'agitation, le produit cristallisé apparaît. Ce produit est filtré sur Büchner, séché puis analysé par diffraction des rayons X sur poudres.

### Exemple 8b

On dissout 0,19 g (soit 3,4 mmol) de potasse dans un mélange de solvants composé de 4 mL de méthanol et de 1,5 mL d'éthylène glycol, puis 0,98 g (soit 2,8 mmol) de ténatoprazole acide de configuration S sont ajoutés à la solution. La dissolution est rapide. Après quelques heures d'agitation à 0°C, le produit cristallisé apparaît. Ce produit est filtré sur Büchner, séché puis analysé par diffraction des rayons X sur poudres.

### Exemple 8c

On dissout 0,19 g (soit 3,4 mmol) de potasse dans un mélange de solvants composé de 5 mL de dioxane et de 2 mL d'éthylène glycol, puis 1,1 g (soit 3,2 mmol) de ténatoprazole acide de configuration S sont ajoutés à la solution. La dissolution est rapide. Après quelques heures d'agitation à 0°C, le produit cristallisé apparaît. Ce produit est filtré sur Büchner, séché puis analysé par diffraction des rayons X sur poudres.

### Exemple 9

### Dédoublement du solvate d'éthanol du sel de potassium du ténatoprazole par cristallisation préférentielle, à l'échelle de 50 cc dans l'éthanol.

### • Conditions associées à l'équilibre

Le tableau ci-dessous montre la solubilité du mélange racémique du sel de potassium solvaté par l'éthanol dans une solution de potasse éthanolique tel que l'excès de potasse soit d'environ 0,2 équivalent molaire par rapport au sel de potassium.

| | | |
|---|---|---|
| Température (°C) | 33,9 | 37 |
| Solubilité (% massique) | 4,24 | 5,3 |

### • Variation de T_{HOMO} avec l'excès énantiomérique

Les résultats montrés dans le tableau suivant correspondent aux étapes (iii) à (v).

| | | | | | |
|---|---|---|---|---|---|
| excès énantiomérique (% ee) | 0 | 3 | 6 | 9 | 11 |
| T_{HOMO} (°C) | T_{L}=33,9 | 34,8 | 35,2 | 35,5 | 35,7 |

### • Conditions associées à la cinétique

Les conditions ci-dessous sont déterminées pour être utilisées lors des étapes (vi) à (ix)

Température T_{I} = 38°C > T_{HOMO} = 35,7°C

Température d'ensemencement = T_{E} = 30°C

Température T_{F} = 19°C

Rampe de température = T = f(t) = 38-2/3*t (t en minutes) suivie par un plateau à 19°C.

| | | | |
|---|---|---|---|
| Température (°C) | 38 | 19 | 19 |
| t (min) | 0 | 30 | T_{Filtration} |

### • Conditions Initiales

Excès énantiomérique = 11%

| Masse d'éthanol | Masse de ténatoprazole acide (racémique) | Masse de ténatoprazole acide (configuration S) | Masse de KOH |
|---|---|---|---|
| 40 g | 1,02 g | 0,125 g | 0,194 g |
| / | 2,95 mmol | 0,36 mmol | 3,46 mmol |

### • Résultats

| No. | Masse d'antipode pur (g)* | Pureté optique (% ee) |
|---|---|---|
| 1 | 0,57 | -98,5 |
| 2 | 0,8 | +91,05 |
| 3 | 0,60 | -89 |
| 4 | 0,50 | +90,7 |
| 5 | 0,58 | -90,6 |
| 6 | 0,62 | +93,3 |
| **Moyenne** | **0,61** | **92,2** |

| | | |
|---|---|---|
| * Compte tenu de l'efflorescence des solvates d'éthanol des sels de potassium : le sel de potassium énantiomériquement pur utilisé pour ensemencer le système se présente sous forme d'une suspension ; le produit récolté après filtration sur Büchner subit une conversion rapide et totale en dihydrate qui correspond à la masse pesée. | | |

### Exemple 10

### Dédoublement du solvate de méthanol du sel de potassium du ténatoprazole par cristallisation préférentielle, à l'échelle de 250 cc dans le méthanol.

### • Conditions associées à l'équilibre

Le tableau ci-dessous montre la solubilité du mélange racémique du sel de potassium solvaté par le méthanol dans une solution de potasse méthanolique tel que l'excès de potasse soit d'environ 0,2 équivalent molaire par rapport au sel de potassium.

| | | | | | |
|---|---|---|---|---|---|
| Température (°C) | 30 | 38 | 40 | 42 | 56 |
| Solubilité (% massique) | 4,4 | 5,2 | 6,5 | 7,7 | 12,2 |

### • Variation de T_{HOMO} avec l'excès énantiomérique

Les résultats montrés dans le tableau suivant correspondent aux étapes (iii) à (v)

| | | | | | |
|---|---|---|---|---|---|
| excès énantiomérique (% ee) | 0 | 1,85 | 3,69 | 6,26 | 9,9 |
| T_{HOMO} (°C) | 36 | 36,3 | 37 | 37,8 | 38,9 |

### • Conditions associées à la cinétique

Les conditions ci-dessous sont déterminées pour être utilisées lors des étapes (vi) à (ix).

Température T_{I} = 40 > T_{HOMO} = 38.9°C

Température T_{E} = 30°C

Température T_{F} = 20°C

Rampe de température = T = f(t) = 40-1/2*t (t en minutes) suivie par un plateau à 20°C.

| | | | |
|---|---|---|---|
| Température (°C) | 40 | 20 | 20 |
| t (min) | 0 | 40 | T_{Filtration} |

### • Conditions initiales

Excès énantiomérique = 10%

| Masse de méthanol | Masse de ténatoprazole acide (racémique) | Masse de ténatoprazole acide (configuration S) | Masse de KOH |
|---|---|---|---|
| 250 g | 8,31 g | 0,83 g | 1,81 g |
| / | 24,0 mmol | 2,4 mmol | 32,3 mmol |

• Résultats

| No. | Masse d'antipode pur (g)* | Pureté Optique (%) |
|---|---|---|
| 1 | 1,69 | -70,9 |
| 2 | 1,38 | +53,3 |
| 3 | 1,7 | -57,1 |
| **Moyenne** | **1,59** | **60,4** |

| | | |
|---|---|---|
| * Compte tenu de l'efflorescence des solvates d'éthanol des sels de potassium : le sel de potassium énantiomériquement pur utilisé pour ensemencer le système se présente sous forme d'une suspension ; le produit récolté après filtration sur Büchner subit une conversion rapide et totale en dihydrate qui correspond à la masse pesée. | | |

### Exemple 11

### Dédoublement du solvate d'éthylène glycol du sel de potassium du ténatoprazole par cristallisation préférentielle à l'échelle de 200 cc dans un mélange éthanol/éthylène glycol (80% VV-20%VV).

### • Conditions associées à l'équilibre

Le tableau ci-dessous montre la solubilité du mélange racémique du sel de potassium solvaté par l'éthylène glycol dans le mélange éthanol/éthylène glycol (80% V-20%V) avec un excès de potasse tel que l'excès de potasse soit d'environ 0,2 équivalent molaire par rapport au sel de potassium.

| | | | | |
|---|---|---|---|---|
| Température (°C) | 28 | 32 | 34,5 | 39 |
| Solubilité (% massique) | 20,3 | 24 | 25,6 | 33,5 |

### • Variation de T_{HOMO} avec l'excès énantiomérique

Les résultats montrés dans le tableau suivant correspondent aux étapes (iii) à (v).

| | | | | | |
|---|---|---|---|---|---|
| excès énantiomérique (% ee) | 0 | 3 | 6 | 9 | 10,7 |
| T_{HOMO} (°C) | 34,4 | 34,9 | 35,4 | 35,9 | 36,2 |

### • Conditions associées à la cinétique

Les conditions ci-dessous sont déterminées pour être utilisées lors des étapes (vi) à (ix).

Température T_{I} = 40 > T_{HOMO} = 36,2°C

Température T_{E} = 30°C

Température T_{F} = 20°C

Rampe de température = T = f(t) = 40-1/2*t (t en minutes) suivie par un plateau à 20°C.

| | | | |
|---|---|---|---|
| Température (°C) | 40 | 20 | 20 |
| t (min) | 0 | 40 | T_{Filtration} |

### • Conditions initiales

| Masse d'éthanol | Masse d'éthylène glycol | Masse de ténatoprazole acide (racémique) | Masse de ténatoprazole acide (configuration S) | Masse de KOH |
|---|---|---|---|---|
| 115 g | 47 g | 40,9 g | 4,9 g (10,7 %ee) | 8 g |
| / | / | 118,2 mmol | 14,2 mmol | 142,6 mmol |

### • Résultats

| No. | Masse d'antipode pur (g) | Pureté optique (%) |
|---|---|---|
| 1 | 10,4 | - 62,9 |
| 2 | 13,9 | + 78,9 |
| 3 | 12,9 | - 81,9 |
| **Moyenne** | **12,4** | **74,6** |

### EXEMPLE 12

### Dédoublement du solvate d'éthylène glycol du sel de potassium du ténatoprazole par cristallisation préférentielle à l'échelle de 2 litres dans un mélange éthanol/éthylène glycol (80% VV-20%VV).

### • Variation de T_{HOMO} avec l'excès énantiomérique

Les résultats montrés dans le tableau suivant correspondent aux étapes (iii) à (v).

| | | |
|---|---|---|
| excès énantiomérique (% ee) | 0 | 9 |
| T_{HOMO} (°C) | TL = 39,1 | 41,8 |

### • Conditions associées à la cinétique

Les conditions ci-dessous sont déterminées pour être utilisées lors des étapes (vi) à (ix).

Température T_{I} = 45°C > T_{HOMO} = 41,8°C

Température T_{E} = 32°C

Température T_{F} = 25°C

Rampe de température = T = f(t) = 45-1/2*t (t en minutes) suivie par un plateau à 25°C.

Agitation mécanique à 500 rpm, à l'aide d'une pale d'agitation à double hélice.

| | | | |
|---|---|---|---|
| Température (°C) | 45 | 25 | 25 |
| t(min) | 0 | 40 | T_{Filtration} |

### • Conditions Initiales

| Masse d'éthanol | Masse d'éthylène glycol | Masse (racémique) | Masse (configuration S) | Masse de KOH |
|---|---|---|---|---|
| 1200 g | 300 g | 600 g | 85 g (12,4 %ee) | 135 g |
| / | / | 1,73 mol | 0,25 mol | 2 , 4 mol |

### • Résultats

| No. | Masse d'antipode pur (g) | Pureté optique (%) |
|---|---|---|
| 1 | 214,4 | +67 |
| 2 | 111,6 | -93 |
| 3 | 88,7 | +51 |
| **Moyenne** | **138,2** | **70** |

La filtration a lieu par centrifugation à 5000 rpm avec une chaussette de diamètre 200 mm, de hauteur 100 mm et dont le diamètre des pores du média filtrant en nylon est de 20 µm.

### Exemple 13

### Recristallisation d'une récolte dans différents solvants

Les exemples 13a et 13b décrivent la recristallisation du mélange des récoltes dans différents solvants obtenues selon le procédé décrit dans l'exemple 12. Seules les récoltes permettant d'obtenir l'énantiomère (-) ont été utilisées.

### Exemple 13a

On recristallise 100 g de sel de potassium dihydraté du ténatoprazole de pureté optique = 74% ee, dans 1600 g d'éthanol en présence de 2,6 g de KOH. Après dissolution totale à 55°C, on recristallise par diminution de la température jusqu'à 17°C, puis la suspension est filtrée sur Büchner après vérification du pouvoir rotatoire de la liqueur mère. La masse de la récolte est de 64 g du dihydrate du sel de potassium du ténatoprazole avec une pureté optique supérieure à 99%ee.

### Exemple 13b

On recristallise 100 g de sel de potassium dihydraté du ténatoprazole de pureté optique = 74% ee, dans 1200 g de méthanol en présence de 2,6 g de KOH. Après dissolution totale à 50°C, on recristallise par diminution de la température jusqu'à 10°C, puis la suspension est filtrée sur Büchner après vérification du pouvoir rotatoire de la liqueur mère. La masse de la récolte est de 57 g du dihydrate du sel de potassium du ténatoprazole avec une pureté optique supérieure à 99% ee.

### Exemple 14

### Structures cristallines

Les exemples 14a et 14b présentent les structures cristallines de deux composés énantiomériquement purs obtenus après la mise en oeuvre du procédé de dédoublement par cristallisation préférentielle tel que décrit respectivement dans les exemples 9 et 11 et 12.

Les intensités de diffraction ont été mesurées avec un diffractomètre SMART APEX automatique (BRUKER).

La structure a été résolue avec les logiciels Saintplus, Sadabs et Shelxs.

### Exemple 14a

Le monocristal du solvate d'éthanol énantiomériquement pur a été obtenu par évaporation très lente d'une solution saturée en sel de potassium du ténatoprazole. Les caractéristiques de cette phase sont les suivantes :
- Monoclinique P2₁
- a = 10,98 Å, b = 7,7 Å, c = 14,03 Å
- α = 90,0°, β = 101,5°, γ # 90,0°

La maille cristalline élémentaire contient 2 molécules d'éthanol dans l'unité asymétrique.

La valeur du paramètre de Flack est 0,07(3), ce qui permet de conclure que la molécule a la configuration absolue S.

### Exemple 14b

Le monocristal du solvate d'éthylène glycol énantiomériquement pur a été obtenu par évaporation très lente d'une solution saturée en sel de potassium du ténatoprazole. Les caractéristiques de cette phase sont les suivantes :
- Orthorhombique P2₁2₁2₁
- a = 7,96 Å, b = 10,0 Å, c = 25,78 Å
- α = β = γ = 90,0°

La maille cristalline élémentaire contient 1 molécule d'éthylène glycol dans l'unité asymétrique.

La valeur du paramètre de Flack est 0,07(5), ce qui permet de conclure que la molécule a la configuration absolue S. La valeur du pouvoir rotatoire mesuré dans l'éthanol à λ = 546 nm est de -1,01°.

### Exemple 15

On dissout 0.29 g (soit 5,2 mmol) de potasse (KOH) dans 10 mL d'alcool isopropylique, puis on ajoute 1,52 g (soit 4,4 mmol) de ténatoprazole acide racémique. Après cristallisation à 0°C, le produit cristallisé est filtré sur Büchner et analysé par diffraction des RX sur poudres.

Le produit obtenu correspond au sel de potassium du ténatoprazole racémique monohydraté cristallisant sous forme de composé racémique.

### Exemple 16

On dissout 0,18 g (soit 3,2 mmol) de potasse (KOH) et 0,96 g (soit 2,8 mmol) de ténatoprazole acide racémique dans un mélange de solvant constitué par 10 mL d'éthanol et 2 mL de propylène glycol. Le produit cristallisé est filtré sur Büchner et analysé par diffraction des RX sur poudres.

Le produit obtenu correspond au sel de potassium du ténatoprazole racémique monohydraté cristallisant sous forme de composé racémique.

### Exemple 17

On dissout 0,14 g (soit 2,6 mmol) de potasse (KOH) dans un mélange de solvant constitué par 14 mL d'éthanol et 0,4 mL d'eau, puis 0,74 g (soit 2,1 mmol) de ténatoprazole acide racémique sont ajoutés à la solution. La dissolution est rapide. Après quelques heures d'agitation à 10°C, le produit cristallisé apparaît. Ce produit est filtré sur Büchner puis recristallisé dans le 1,4-dioxane. Le produit issu de cette recristallisation est filtré sur Büchner, séché puis analysé par diffraction des RX sur poudres.

Le produit obtenu correspond au sel de potassium du ténatoprazole énantiomériquement pur monohydraté cristallisant sous forme de composé racémique.

### Exemple 18

On dissout 0,48 g (soit 8,6 mmol) de potasse (KOH) dans un mélange de solvant constitué par 10 mL d'eau et 1,9 mL de N-méthylpyrrolidinone, puis 2,46 g (soit 7,1 mmol) de ténatoprazole acide racémique sont ajoutés à la solution. La dissolution est rapide. Après quelques heures d'agitation à 10°C, le produit cristallisé apparaît. Ce produit est filtré sur Büchner, séché puis analysé par diffraction des RX sur poudres. Le produit obtenu correspond à un composé racémique du sel de potassium hétérosolvaté par l'eau et la N-méthylpyridinone.

### Exemple 19

### Transformation d'un sel de potassium en sel de sodium

Le sel de potassium dihydraté énantiomériquement pur (9,6 g) est dissous dans l'eau (50 mL). Le sel étant à solubilité non congruente, il se dissocie partiellement. De l'acide acétique (50 mL, 0,5 mol/L) est ajouté afin de neutraliser la base et de cristalliser l'acide libre. Puis, la phase cristallisée est récupérée par filtration, et rincée abondamment par de l'eau afin d'éliminer l'acide acétique et le potassium.

La masse d'acide obtenu (S-(-)-ténatoprazole) est de 6,57 g soit un rendement d'environ 83%

Dans un ballon de 50 ml, on introduit 1,0 g de S-(-)-ténatoprazole obtenu comme indiqué ci-dessus, sous agitation modérée et à température ambiante, on ajoute 1,0 mL d'eau puis 0,6 mL d'hydroxyde de sodium en solution aqueuse (5 M).

On chauffe le mélange réactionnel à 60°C pendant 2,5 heures sous agitation pour obtenir un liquide huileux qui est refroidi à température ambiante. Le solvant est éliminé sous pression réduite à 40°C dans un évaporateur rotatif. Après addition de 6 mL d'acétone, sous agitation, on recueille par filtration sur verre fritté un produit de couleur jaune pâle que l'on rince avec 2,0 mL d'acétone.

Après séchage à 40°C sous pression réduite pendant 20 h, on obtient 1,1 g de sel de sodium monohydraté du S-ténatoprazole dont la caractérisation est faite par analyse thermique et par diffraction X.

## Revendications

1. Utilisation de sels de potassium du ténatoprazole, représentés par la formule générale suivante, 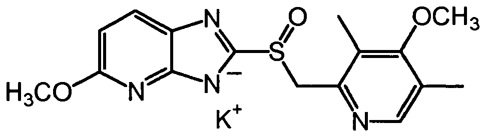 constitués d'un mélange équimolaire d'énantiomères de configuration R et S cristallisant sous forme de conglomérat, lesdits sels étant choisis parmi le sel de potassium dihydraté du ténatoprazole, le diméthanolate de potassium du ténatoprazole, le diéthanolate de potassium du ténatoprazole et l'éthylène-glycolate de potassium du ténatoprazole, pour la préparation d'un sel de sodium du ténatoprazole énantiomériquement pur, par dédoublement des énantiomères optiques d'un des sels racémiques de potassium correspondants, selon un procédé de cristallisation préférentielle, puis transformation de l'un desdits énantiomères optiques du sel de potassium en l'énantiomère correspondant du sel de sodium.

2. utilisation selon la revendication 1, **caractérisé en ce qu'**il s'agit du sel de potassium dihydraté du ténatoprazole, ledit sel produisant un spectre de diffraction aux rayons X comprenant des raies aux distances inter-réticulaires suivantes : 15,16 ; 8,07 ; 7,59 ; 7,23 ; 7,02 ; 5,72 ; 5,55 ; 5,41 ; 5,32 ; 5,05 ; 4,51 ; 4,35 ; 4,29 ; 4,22 ; 4,061 ; 3,926 ; 3,748 ; 3,624 ; 3,539 ; 3,487 ; 3,436 ; 3,392 ; 3,292 ; 3,252 ; 3,222 ; 3,102 ; 3,034 (en Angströms).

3. Utilisation selon la revendication 1, **caractérisé en ce qu'**il s'agit du diméthanolate de potassium du ténatoprazole, ledit sel produisant un spectre de diffraction aux rayons X comprenant des raies aux distances inter-réticulaires suivantes : 13,79 ; 6,87 ; 5,84 ; 5,44 ; 4,83 ; 4,57 ; 4,31 ; 3,97 ; 3,921 ; 3,865 ; 3,794 ; 3,644 ; 3,56 ; 3,508 ; 3,431 ; 3,393 ; 3,309 ; 3,233 ; 3,116 (en Angströms).

4. Utilisation selon la revendication 1, **caractérisé en ce qu'**il s'agit du diéthanolate de potassium du ténatoprazole, ledit sel produisant un spectre de diffraction aux rayons X comprenant des raies aux distances inter-réticulaires suivantes : 13,79 ; 10,73 ; 9,41 ; 7,75 ; 6,87 ; 6,71 ; 6, 40 ; 6,25 ; 5, 97 ; 5, 463 ; 5,377 ; 5,128 ; 4,918 ; 4,711 ; 4,562 ; 4,418 ; 4,020 ; 3,936 ; 3,892 ; 3,855 ; 3,705 ; 3,656 ; 3,623 ; 3,573 ; 3,508 ; 3,460 ; 3,446 ; 3,363 ; 3,302 ; 3,249 ; 3,167 ; 3,129 ; 3,102 ; 3,043 (en Angströms).

5. Utilisation selon la revendication 1, **caractérisé en ce qu'**il s'agit de l'éthylène glycolate de potassium du ténatoprazole, ledit sel produisant un spectre de diffraction aux rayons X comprenant des raies aux distances inter-réticulaires suivants 12,92 ; 9,30 ; 7,89 ; 7,59 ; 6,77 ; 6,05 ; 5,828 ; 5,604 ; 5,409 ; 5,026 ; 4,907 ; 4,657 ; 4,569 ; 4,478 ; 4,295 ; 4,178 ; 4,013 ; 3,972 ; 3,931 ; 3,798 ; 3,692 ; 3,650 ; 3,608 ; 3,543 ; 3,396 ; 3,340 ; 3,300 ; 3,271 ; 3,204 ; 3,148 ; 3,105 ; 3,060 ; 3,013 (en Angströms).

6. Utilisation selon la revendication 1, pour la préparation du sel de sodium du S-ténatoprazole.

7. Utilisation selon la revendication 1, pour la préparation du sel de sodium du R-ténatoprazole.

## Patentansprüche

1. Einsatz von Kaliumsalzen des Tenatoprazols, die der nachfolgenden allgemeinen Formel entsprechen: 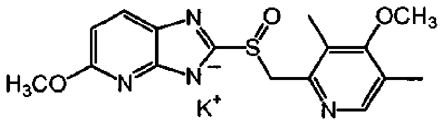 bestehend aus einer äquimolaren Mischung aus Enantiomeren der Konfiguriation R und S, die sich in Form eines Konglomerats kristallisieren, wobei die Salze aus der Gruppe vom dehydratierten Kaliumsalz des Tenatoprazols, Kaliumdimethanolat des Tenatoprazols, Tenatoprazol-Kaliumdiethanolat und Tenatoprazol-Ethylenglykolat, zur Herstellung eines enantiomer reinen Tenatoprazol-Natriumsalzes im Wege der Duplikation der optischen Enantiomere eines der entsprechenden racemischen Kaliumsalze in einem bevorzugten Kristallisationsverfahren, gefolgt durch die Umwandlung eines dieser optischen Enantiomeren des Kaliumsalzes in das entsprechende Enantiomer des Natriumsalzes.

2. Einsatz nach Anspruch 1, **dadurch gekennzeichnet, dass** es sich um das dehydratierte Kaliumsalz des Tenatoprazols handelt, wobei das Salz ein Röntgen-Beugungsspektrum aufweist, das Linien in den nachfolgenden interplanären Abständen umfasst: 15,16; 8,07; 7,59; 7,23; 7,02; 5,72; 5,55; 5,41; 5,32; 5,05; 4,51; 4,35; 4,29; 4,22; 4,061; 3,926; 3,748; 3,624; 3,539; 3,487; 3,436; 3,392; 3,292; 3,252; 3,222; 3,102; 3,034 (in Angström).

3. Einsatz nach Anspruch 1, **dadurch gekennzeichnet, dass** es sich um das Kaliumdimethanolat des Tenatoprazols, wobei das Salz ein Röntgen-Beugungsspektrum aufweist, das Linien in den nachfolgenden interplanären Abständen umfasst: 13,79; 6,87; 5,84; 5,44; 4,83; 4,57; 4,31; 3,97; 3,921; 3,865; 3,794; 3,644; 3,56; 3,508; 3,431; 3,393; 3,309; 3,233; 3,116 (in Angström).

4. Einsatz nach Anspruch 1, **dadurch gekennzeichnet, dass** es sich um das Kaliumdiethanolat des Tenatoprazols handelt, wobei das Salz ein Röntgen-Beugungsspektrum aufweist, das Linien in den nachfolgenden interplanären Abständen umfasst: 13,79; 10,73; 9,41; 7,75; 6,87; 6,71; 6,40; 6,25; 5,97; 5,463; 5,377; 5,128; 4,918; 4,711; 4,562; 4,418; 4,020; 3,936; 3,892; 3,855; 3,705; 3,656; 3,623; 3,573; 3,508; 3,460; 3,446; 3,363; 3,302; 3,249; 3,167; 3,129; 3,102; 3,043 (in Angström).

5. Einsatz nach Anspruch 1, **dadurch gekennzeichnet, dass** es sich um das Kaliumethylenglykolat des Tenatoprazols handelt, wobei das Salz ein Röntgen-Beugungsspektrum aufweist, das Linien in den nachfolgenden interplanären Abständen umfasst: 12,92; 9,30; 7,89; 7,59; 6,77; 6,05; 5,828; 5,604; 5,409; 5,026; 4,907; 4,657; 4,569; 4,478; 4,295; 4,178; 4,013; 3,972; 3,931; 3,798; 3,692; 3,650; 3,608; 3,543; 3,396; 3,340; 3,300; 3,271; 3,204; 3,148; 3,105; 3,060; 3,013 (in Angström)

6. Einsatz nach Anspruch 1 zur Herstellung des S-Tenatoprazol-Natriumsalzes.

7. Einsatz nach Anspruch 1 zur Herstellung des R-Tenatoprazol-Natriumsalzes.

## Claims

1. The use of tenatoprazole potassium salts represented by the following general formula: 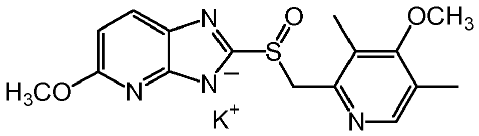 formed from an equimolar mixture of enantiomers of R and S configuration, crystallizing in the form of conglomerates, said salts being selected from potassium tenatoprazole dihydrate, potassium tenatoprazole dimethanolate, potassium tenatoprazole diethanolate and potassium tenatoprazole ethylene glycolate, for the preparation of an enantiomerically pure tenatoprazole sodium salt, by resolution of the optical enantiomers of one of the corresponding racemic potassium salts, according to a process of preferential crystallization, followed by conversion of one of said optical enantiomers of the potassium salt into the corresponding enantiomer of the sodium salt.

2. The use according to claim 1, **characterized in that** it is tenatoprazole potassium salt dihydrate, said salt producing an X-ray diffraction spectrum comprising lines at the following inter-reticular distances: 15.16; 8.07; 7.59; 7.23; 7.02; 5.72; 5.55; 5.41; 5.32; 5.05; 4.51; 4.35; 4.29; 4.22; 4.061; 3.926; 3.748; 3.624; 3.539; 3.487; 3.436; 3.392; 3.292; 3.252; 3.222; 3.102; 3.034 (in Angströms).

3. The use according to claim 1, **characterized in that** it is potassium tenatoprazole dimethanolate, said salt producing an X-ray diffraction spectrum comprising lines at the following inter-reticular distances: 13.79; 6.87; 5.84; 5.44; 4.83; 4.57; 4.31; 3.97; 3.921; 3.865; 3.794; 3.644; 3.56; 3.508; 3.431; 3.393; 3.309; 3.233; 3.116 (in Angströms).

4. The use according to claim 1, **characterized in that** it is potassium tenatoprazole diethanolate, said salt producing an X-ray diffraction spectrum comprising lines at the following inter-reticular distances: 13.79; 10.73; 9.41; 7.75; 6.87; 6.71; 6.40; 6.25; 5.97; 5.463; 5.377; 5.128; 4.918; 4.711; 4.562; 4.418; 4.020; 3.936; 3.892; 3.855; 3.705; 3.656; 3.623; 3.573; 3.508; 3.460; 3.446; 3.363; 3.302; 3.249; 3.167; 3.129; 3.102; 3.043 (in Angströms).

5. The use according to claim 1, **characterized in that** it is potassium tenatoprazole ethylene glycolate, said salt producing an X-ray diffraction spectrum comprising lines at the following inter-reticular distances: 12.92; 9.30; 7.89; 7.59; 6.77; 6.05; 5.828; 5.604; 5.409; 5.026; 4.907; 4.657; 4.569; 4.478; 4.295; 4.178; 4.013; 3.972; 3.931; 3.798; 3.692; 3.650; 3.608; 3.543; 3.396; 3.340; 3.300; 3.271; 3.204; 3.148; 3.105; 3.060; 3.013 (in Angströms).

6. The use according to claim 1, for the preparation of S-tenatoprazole sodium salt.

7. The use according to claim 1, for the preparation of R-tenatoprazole sodium salt.
